# EUROPEAN PATENT APPLICATION

(11) **EP 2 149 357 A1**
(43) Date of publication of application: **03.02.2010**
(21) Application number: 08753055.6
(22) Date of filing: 21.05.2008
(51) Int. Cl.: A61F 13/15, A61F 13/00, A61F 13/472

(54) **ABSORPTIVE ARTICLE**

(30) Priority: 22.05.2007 JP 2007135237
(71) Applicant: Unicharm Corporation, Ehime 799-0111 (JP)
(72) Inventor: NISHIKAWA, Kumiko, Kanonji-shi Kagawa 769-1602 (JP); NODA, Yuki, Kanonji-shi Kagawa 769-1602 (JP); KURODA, Kenichiro, Kanonji-shi Kagawa 769-1602 (JP)
(74) Representative: Sperling, Rüdiger
(86) International application number: PCT/JP2008/059378
(87) International publication number: WO 2008/143298

(57) **Abstract**

An absorptive article that has increased flexibility and in which separation of a sheet is prevented. The absorptive article that is longer than is wide has a front surface sheet at least a portion of which is liquid pervious, a liquid impervious rear surface sheet, an absorptive body having liquid retaining properties and placed between the front and rear surface sheets, two side sheets arranged on both sides of the absorptive body such that at least a part of each of the two side sheets covers the front surface sheet, and embossed sections having compressed sections respectively formed along the two side sheets. The embossed sections are each composed of first embossed sections formed on the front edge side and the rear edge side of the absorptive article that are end sections in the longitudinal direction of the absorptive article, and of a second embossed section formed at the center in the longitudinal direction of the absorptive article. The first embossed sections are wider in the lateral direction than the second embossed sections, and each of the compression sections structuring the second embossed sections is formed separated from each other.

## Description

### TECHNICAL FIELD

The present invention relates to an absorbent article, especially to an absorbent article of superior flexibility.

### BACKGROUND ART

Sanitary napkins, panty liners, urine-absorbing pads and the like have conventionally been used as an absorbent article for absorbing discharges such as body fluids. These absorbent articles have: an absorbent body for absorbing and retaining body fluid and the like; a liquid permeable surface sheet for covering the surface on a skin contacting side of the absorbent body, and a liquid impermeable back sheet for covering the skin noncontacting side of the absorbent body positioned on the clothing side.

Absorbent articles used by being fixed to a crotch piece of underwear, such as sanitary napkins, can be easily deformed by motions of the wearer, especially by movements of the legs. To prevent side leakage due to the deformation, an absorbent article with side sheets provided in the side edge portions of an absorbent body is provided. The side sheets are joined thereto by placing them on a surface sheet or a back sheet and joining by a joining method such as heat-sealing, heat-embossing and the like.

Regarding the abovementioned absorbent article, for example, Japanese Unexamined Patent Application No. 2003-243834 (hereinafter referred to as Patent Document 1) discloses a sanitary napkin having a joining portion formed continuously in a longitudinal direction thereof, which joins the abovementioned sheets and an absorbent body, and has narrow-width portions and wide-width portions.

However, the sanitary napkin disclosed in Patent Document 1 has a joining portion formed continuously in a longitudinal direction. This may allow the sanitary napkin to bend irregularly, since the sanitary napkin does not have a folding line for allowing the sanitary napkin to bend along the curvature of the wearer's body. This may generate more gaps between the sanitary napkin and the wearer's body, which may lead to a leakage of discharged matter such as menstrual blood.

Additionally, the wide-width portions of the joining portion may have an increased stiffness as a result of an improved delamination strength, which may inhibit the deformation of the sanitary napkin along the curvature of the wearer's body.

In contrast, for example, Japanese Unexamined Patent Application No. 2005-323904 (hereinafter referred to as Patent Document 2) discloses an absorbent article with a plurality of curvilinear embossed patterns formed on side sheets, to prevent stiffness of the joining portions, which improves the flexibility. However, since the embossed patterns are also formed substantially continuously in a longitudinal direction, an optimal flexibility in deformation cannot be provided.

Moreover, in the sanitary napkin disclosed in Patent Document 2, the area and width of the embossed patterns provided thereon are not considered. Regarding absorbent articles such as sanitary napkins, a posterior portion thereof, in other words the region contacting the buttocks, is subjected to a pressure in a width direction due to motions such as walking while wearing. This may lead to an easy peeling of the sheets when a pressure in the width direction is applied to a posterior portion of the product, which may lead to a breakage of the product. Additionally, since the embossed patterns are formed continuously in a longitudinal direction, a single breakage may lead to a wide-ranging breakage in the longitudinal direction. The breakage may allow a leakage of liquid such as menstrual blood.

### DISCLOSURE OF THE INVENTION

### Problems to be Solved by the Invention

The objective of the present invention is to improve the flexibility of a sanitary napkin for an easy deformation, by discontinuously forming compressed portions constituting an embossed portion and to provide an absorbent article which is able to have improved resistance against delamination of laminated sheets, by varying the area of the compressed portions constituting the embossed portions formed in anterior, posterior and central portions of the sanitary napkin.

### Means for Solving the Problems

In order to achieve the abovementioned objective, the present inventors found that an absorbent body can deform flexibly, and delamination and breakup of the product can be prevented by: providing a first embossed portion and a second embossed portion of different size on side sheets disposed on both sides of the absorbent body in which at least a part thereof covers a surface sheet; and spacing compressed portions constituting the first and second embossed portions apart each other, thus leading to the completion of the present invention. Specifically, the present invention provides the following absorbent articles.

In a first aspect of the absorbent article, an elongated shaped absorbent article is provided, including: a surface sheet which is at least partially liquid permeable; a back sheet which is liquid impermeable; a liquid retentive absorbent body which is disposed between the surface sheet and the back sheet; a pair of side sheets which is disposed on both sides of the absorbent sheet so that at least a part thereof covers the surface sheet; and an embossed portion having a plurality of compressed portions formed along the pair of side sheets, in which the embossed portion has a first embossing portion which is formed in both edge portions in a longitudinal direction of the absorbent article, in other words on a front edge side and a rear edge side, and a second embossing portion which is formed in a central portion in the longitudinal direction; the first embossed portion has a width in a width direction orthogonal to the longitudinal direction greater than a width of the second embossed portion; and compressed portions constituting the second embossed portion are formed so as to be spaced apart from each other.

In a second aspect of the absorbent article as described in the first aspect of the present invention, the absorbent article according to the first aspect is provided, in which the second embossed portion has the plurality of compressed portions and a plurality of non-compressed portions formed between each of the compressed portions formed so as to be spaced apart from each other; and a stiffness of each of the plurality of compressed portions is greater than a stiffness of the plurality of non-compressed portions.

In a third aspect of the absorbent article as described in the first or the second aspect of the present invention, in which each of the plurality of compressed portions constituting the second embossed portion has a crossover region in the width direction with a neighboring compressed portion.

In a fourth aspect of the absorbent article as described in any of the first to the third aspect of the present invention, in which an inner edge in the width direction of the second embossed portion is formed outside of an inner edge in the width direction of the first embossed portion.

In a fifth aspect of the absorbent article as described in any of the first to the fourth aspect of the present invention, in which a stiffness of each of the compressed portions constituting the first embossed portion is lower than a stiffness of each of the compressed portions constituting the second embossed portion.

In a sixth aspect of the absorbent article as described in any of the first to the fifth aspect of the present invention, in which a pressure for forming each of a plurality of compressed portions constituting the first embossed portion is lower than a pressure for forming each of a plurality of compressed portions constituting the second embossed portion.

In a seventh aspect of the absorbent article as described in any of the first to the sixth aspect of the present invention, in which the first embossed portion has a plurality of first compressed portions of a length in the width direction substantially same as a length in the width direction of the first embossed portion; and a first auxiliary compressed portion of a length in the width direction shorter than a length in the width direction of the first compressed portion, and the first compressed portion, and the first auxiliary compressed portion are formed alternately in the longitudinal direction.

In an eighth aspect of the absorbent article as described in any of the first to the seventh aspect of the present invention, in which the embossed portion joins the side sheet to the surface sheet.

In a ninth aspect of the absorbent article as described in any of the first to the eighth aspect of the present invention, in which, in a region formed in the second embossed portion in the longitudinal direction, an inner edge in the width direction of the side sheet is configured to be separaable from the surface sheet; and the second embossed portion acts as a folding line for separating the inner edge of the side sheet from the surface sheet.

### Effects of the Invention

The present invention can provide an absorbent article which can flexibly deform an absorbent body, and can prevent delamination of sheets and an absorbent body which constitute the absorbent article.

### BRIEF DESCRIPTION OF THE DRAWINGS

FIG. 1 is a top view of a sanitary napkin according to the first embodiment of the present invention;
FIG. 2 is a cross-sectional view taken along the line X-X in FIG. 1;
FIG. 3 is a drawing illustrating a non-woven fabric used as a surface sheet of a sanitary napkin according to the first embodiment;
FIG. 4 is a partially enlarged view of a sanitary napkin according to the first embodiment;
FIG. 5 is a top view of a sanitary napkin according to the second embodiment;
FIG. 6 is a partially enlarged view of a sanitary napkin according to the second embodiment;
FIG. 7 is a partially enlarged view of a sanitary napkin according to the second embodiment; and
FIG. 8 is a partially enlarged view of a variation of a sanitary napkin according to another embodiment.

### PREFERRED MODE FOR CARRYING OUT THE INVENTION

Embodiments of the present invention are described hereinafter with reference to the accompanying drawings. However, it is to be understood that the embodiments of the present invention are not limited to the following, and the technical scope of the present invention is not limited thereto.

The absorbent articles according to the present invention are worn on the crotch of the human body in order to absorb menstrual blood, urine, and leucorrhea discharged from the human body. Tthe following embodiments are directed to sanitary napkins, the primary object of which is to absorb menstrual blood discharged from the vaginal opening of females. In the following description, one of the two surfaces of the absorbent article, which is directed to the excretory portion, is called "skin contacting side", and the other is called "skin noncontacting side", irrespective of whether clothing is worn.

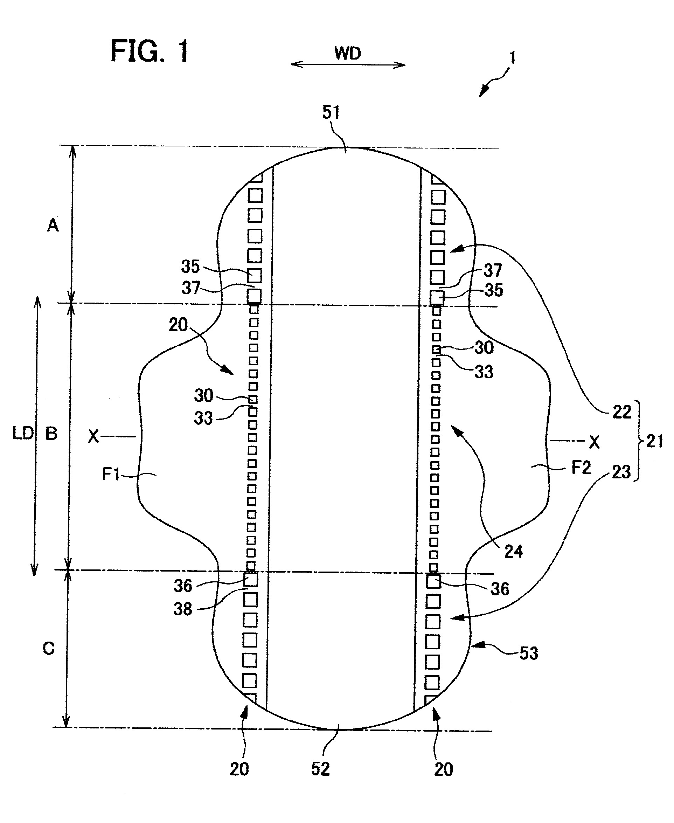

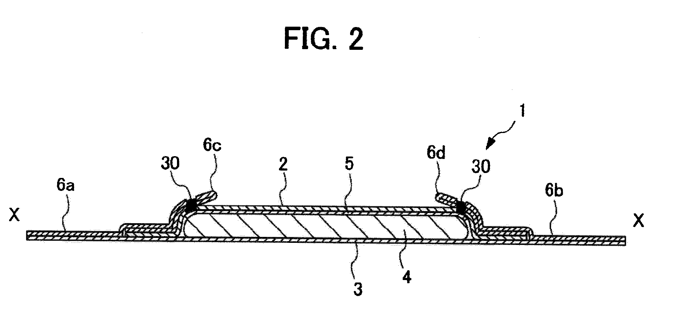

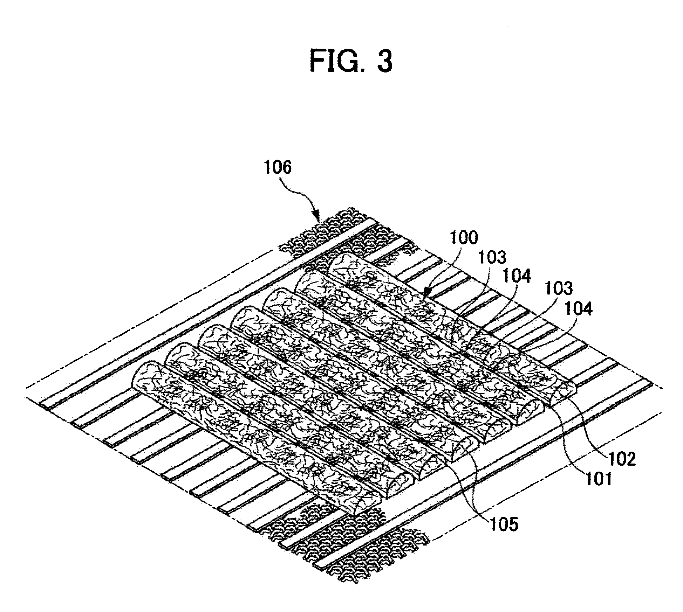

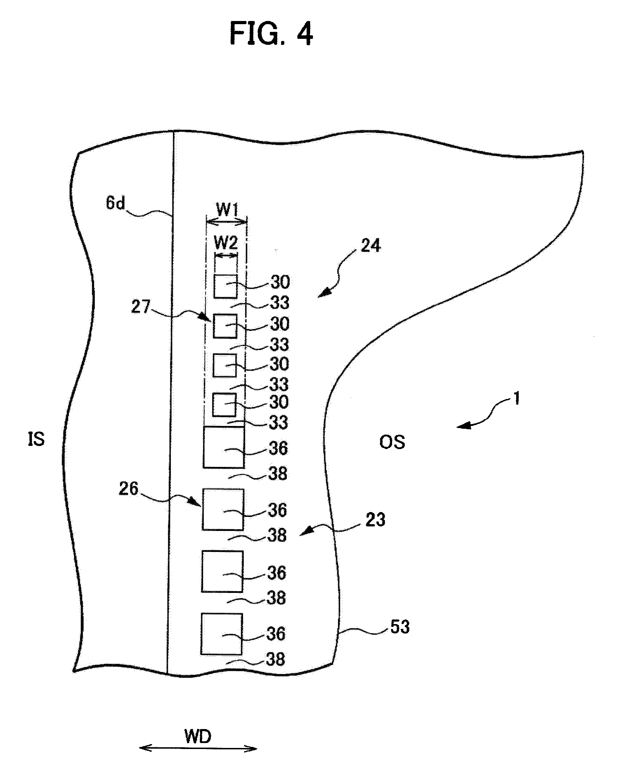

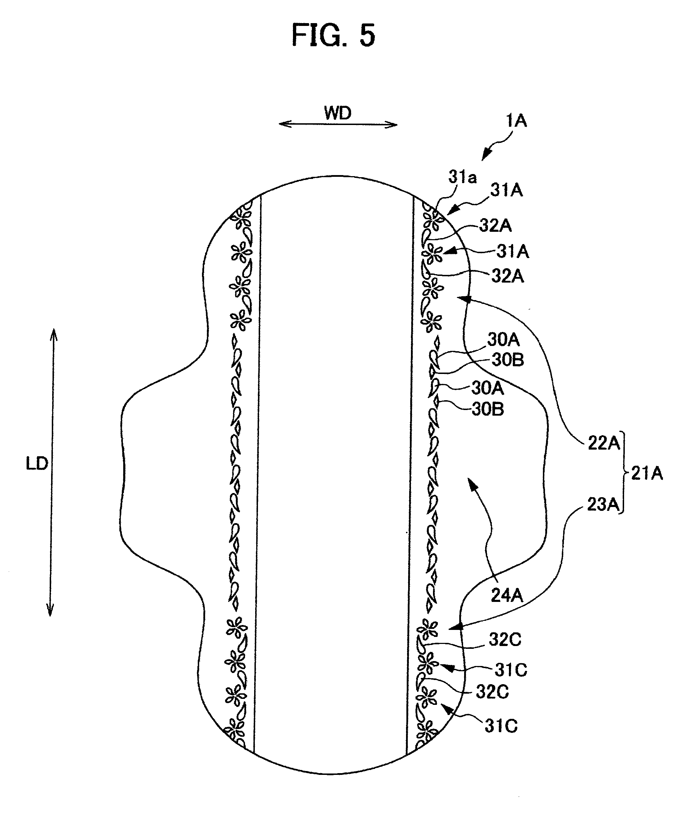

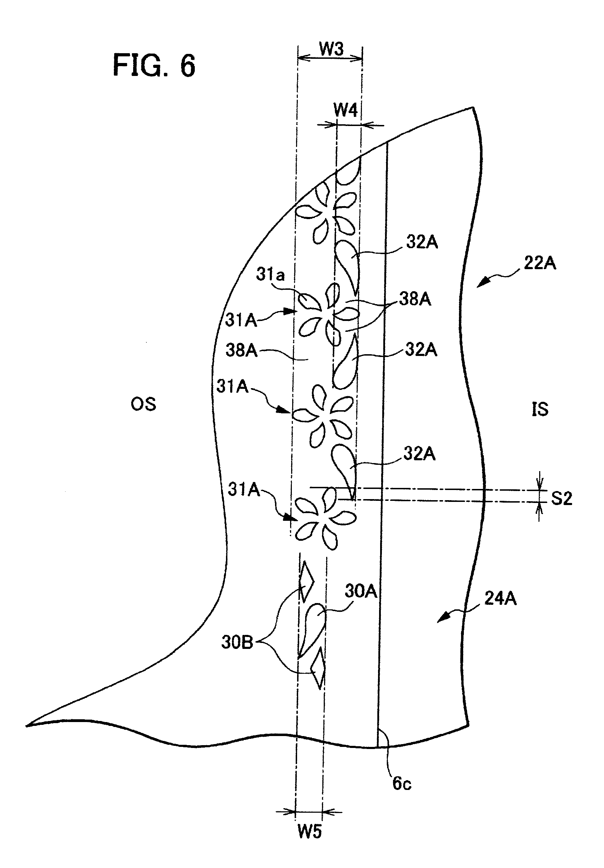

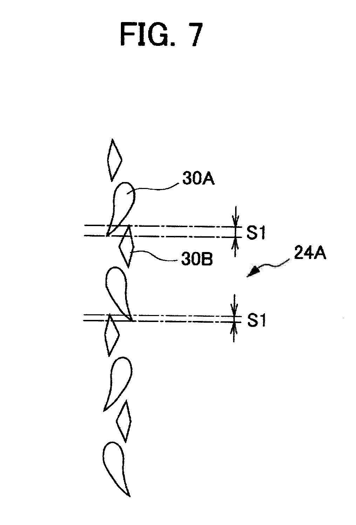

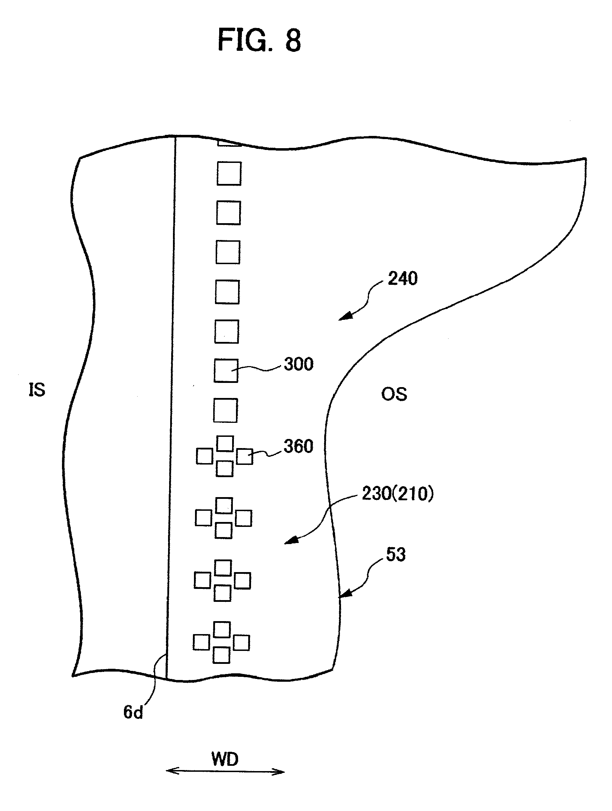

FIG. 1 is a top view of a sanitary napkin according to a first embodiment of the present invention. FIG. 2 is a cross-sectional view taken along the line X-X in Fig. 1. FIG. 3 is a drawing illustrating a non-woven fabric used as a surface sheet of a sanitary napkin according to the first embodiment, and a supporting member used for manufacture thereof. FIG. 4 is a partially enlarged view of an embossed portion of a sanitary napkin according to the first embodiment. FIG. 5 is a top view of a sanitary napkin according to a second embodiment of the present invention. FIG. 6 is a partially enlarged view of a first embossed portion of a sanitary napkin according to the second embodiment. FIG. 7 is a partially enlarged view of a second embossed portion of a sanitary napkin according to the second embodiment. FIG. 8 is a partially enlarged view of a variation of an embossed portion of a sanitary napkin according to another embodiment.

### 1. First Embodiment

The overall configuration of the absorbent article of the present invention will be described with reference to a sanitary napkin 1 according to a first embodiment of the present invention.

### 1.1 General View

As shown in FIG. 1, the sanitary napkin 1 is formed in an elongated shape. A front edge 51 and a rear edge 52 of the sanitary napkin 1 are curvilinear shaped, convexly curved outward in a longitudinal direction. In this specification, the front edge 51 is an edge of the sanitary napkin 1 being worn disposed on an abdomen side of the wearer, and the rear edge 52 is an edge disposed on a back side of the wearer. Additionally, a portion of the sanitary napkin 1 disposed on an abdomen side will be referred to as an anterior portion A, a portion on a back side will be referred to as a posterior portion C, and a portion disposed on a crotch, facing the excretion portion of the wearer, will be referred to as a central portion B. The length of the sanitary napkin 1 in a longitudinal direction is, for example, in the range of 100 to 500 mm, preferably in the range of 150 to 350 mm. The length thereof in a width direction is, for example, preferably in the range of 30 to 200 mm, more preferably in the range of 40 to 180 mm.

As shown in FIG. 2, the sanitary napkin 1 includes: a liquid retentive absorbent body 4; a liquid permeable surface sheet 2 disposed on a skin contacting side of the absorbent body 4, which is one side thereof in a thickness direction; and a liquid impermeable back sheet 3 disposed on a skin noncontacting side of the absorbent body 4, which is another side thereof in the thickness direction. A liquid permeable member such as an intermediate sheet 5 is disposed between the absorbent body 4 and the surface sheet 2. Additionally, the sanitary napkin 1 includes a pair of side sheets disposed on both sides of the absorbent body 4 in a longitudinal direction. The side sheets 6a and 6b are disposed so that at least a part thereof covers the surface sheet 2. These members are not particularly limited and any conventionally known members can be used.

As shown in FIG. 1, the side sheets 6a and 6b include embossed portions 20 having a plurality of compressed portions 30, 35 and 36 formed along a longitudinal direction LD of the side sheets 6a and 6b. The embossed portions 20 include a first embossed portion 21 and a second embossed portion 24. The first embossed portion is composed of an anterior embossed portion 22 formed in the anterior portion A of the sanitary napkin 1 and a posterior embossed portion 23 formed in the posterior portion C. The second embossed portion 24 is formed on the central portion B of the sanitary napkin.

The width of the anterior embossed portion 22 and the posterior embossed portion 23 is greater than the width of the second embossed portion 24. Each of the compressed portions 30, 35 and 36 constituting the embossed portions 20 is formed discontinuously, being spaced apart from each other.

The sanitary napkin 1 includes side flaps F1 and F2 extending in a width direction of the sanitary napkin 1. The side flaps F1 and F2 are provided with an adhesive portion (not shown) on a skin noncontacting side thereof, which allows the sanitary napkin 1 to be fixed to the underwear by fastening the side flaps F1 and F2 being folded back, on an external surface of crotch portion of the underwear. Additionally, an adhesive portion (not shown) extending in a longitudinal direction is provided in a central region in a width direction of a skin noncontacting side of the back sheet 3. The adhesive portion allows the sanitary napkin 1 to be fastened to an internal surface of a crotch portion of the wearer's underwear.

A discharged matter such as menstrual blood discharged from the excretion portion of the wearer passes through the surface sheet 2 and is then absorbed by the absorbent body 4. Since the back sheet 5 disposed on the skin noncontacting side is liquid impermeable, the discharged matter is absorbed by the absorbent body 4 and retained therein, without reaching the skin noncontacting side.

### 1.2 Surface Sheet

The surface sheet 2 is, when being worn, disposed on the wearer's body side, and also brought into contact with the excretory portion. The surface sheet 2 may be entirely or partly liquid permeable, and may be composed of either a single sheet-like member or a plurality of sheet-like members bonded together. In the present embodiment, the surface sheet 2 has a liquid permeable region in a substantially central portion in a width direction of the sanitary napkin 1, and both edges of the surface sheet 2 in the width direction are covered with liquid impermeable side sheets 6a and 6b.

The surface sheet 2 is, as shown in FIG. 3, a non-woven fabric with a plurality of groove portions formed on one side of a non-woven fabric 100, at substantially regular intervals and in parallel with each other. Between the plurality of groove portions 101 arranged at substantially equal intervals, a plurality of convex portions 102 is formed. In each of the groove portions 101, a plurality of openings 103 is formed with a predetermined interval therebetween. Each of the openings 103 is formed to have a substantially circular or a substantially elliptical shape in a planar view. The convex portions 102 are formed in parallel at substantially regular intervals, in the same way as the groove portions 101. The groove portions 101 according to the present embodiment are formed in parallel with each other at substantially regular intervals; however, they are not limited thereto and may be formed at irregular intervals, or may not be in parallel with each other and the intervals between groove portions 101 may vary. In addition, the height of the convex portions 102 may also vary and each of the convex portions 102 may be formed to have different heights.

The groove portions 101 and the openings 103 formed therein are formed by: supporting a fiber web from a lower surface thereof, which is a fiber aggregate, by a net-shaped supporting member 106, which is an air-permeable supporting member; and blowing gas from an upper surface thereof to rearrange the fibers 105 constituting the fiber web. The fiber aggregate is sheet-shaped and has a degree of freedom allowing the fibers 105 to be rearranged. The fiber orientation, sparseness/denseness or the basis weight of the fibers 105 can be adjusted accordingly. By the presence of the groove portions 101 on the surface sheet and of the openings 103 on the groove portions 101, a sanitary napkin having a superior liquid permeability can be obtained. A fiber web formed by the carding method using relatively long fibers is suitable for arranging and rearranging the fibers by blowing an airflow. In order to ensure that the fibers become a non-woven fabric which retains its shape after the groove portions (concave and convex portions) are formed by a plurality of air flows, it is preferable to employ a throw air method where thermoplastic fibers are subjected to hot melting by oven processing (heat treatment). Fibers suitable for this manufacturing method are preferably those having the core-in-sheath structure or side-by-side structure in order to heat-seal intersecting points of the fibers or are more preferably those having the core-in-sheath structure in which sheaths can be infallibly heat-sealed. Specifically, the sheath-core type compound fiber consisting of polyethylene terephthalate and polyethylene, or the sheath-core type compound fiber consisting of polypropylene and polyethylene is preferably used. These fibers may be used alone or in combination. The length of fibers is preferably in the range of 20 to 100 mm, and more preferably in the range of 35 to 65 mm.

A liquid permeable member such as an intermediate sheet 5 can be disposed accordingly between the surface sheet 2 and the absorbent body 4. The intermediate sheet 5 allows the discharged matter passed through the surface sheet 2 to pass therethrough toward the absorbent body 4, while providing cushioning during use. Any conventionally known member such as the same sheet member as that for the surface sheet 2 can be used for the intermediate sheet 5.

### 1.3 Absorbent body

The absorbent body 4 is wrapped in a tissue and absorbs and retains the discharged matter such as menstrual blood passed through the surface sheet 2 and the intermediate sheet 5. Pulverized softwood kraft pulp with 10% of superabsorbency polymer blended thereto, wrapped in a tissue can be used as the absorbent body 4. The basis weight of the absorbent body 4 is, for example, 400 g/m². A tissue of basis weight of 18 g/m² can be used for wrapping the absorbent body 4. It should be noted that, in the present embodiment, a plurality of perforated holes are provided on the tissue, extending in the longitudinal direction of the sanitary napkin 1 and penetrating the tissue in the thickness direction of the sanitary napkin 1.

### 1.4. Back sheet

The back sheet 3 is composed of a liquid impermeable sheet member and prevents the discharged matter and the like retained in the absorbent body 4 from leaking to the skin noncontacting side of the absorbent article. For example, a liquid impermeable polyethylene film of a basis weight of 27 g/m² can be used as the back sheet 3.

### 1.5 Side sheets

A pair of side sheets 6a and 6b is disposed along both edges in the longitudinal direction of the absorbent body 4 so that at least a part thereof covers the surface sheet 2. For example, a through-air non-woven fabric and a polypropylene spunbond non-woven fabric (PPSB) can be used as the side sheets 6a and 6b. The side sheets 6a and 6b are folded in so that their inner ends 6c and 6d are folded lines. One edge of the side sheets 6a and 6b on the skin contacting side extends to an outer edge 53 of the sanitary napkin 1 and another edge on the skin noncontacting side is disposed so as to lap over the surface sheet 2. It should be noted that the side sheets 6a and 6b illustrated in the present invention are folded in on the surface sheet side; however, the side sheets can be joined to an absorbent article without folding in.

### 1.5.1 Embossed portion

As shown in FIG. 1, the embossed portion 20 includes a first embossed portion 21 and a second embossed portion 24. The first embossed portion is composed of an anterior embossed portion 22 formed in the anterior portion A and a posterior embossed portion 23 formed in the posterior portion C. As described later, a plurality of compressed portions 30, 35 and 36 formed along the side sheets 6a and 6b, and non-compressed portions 33, 37, and 38 are formed between each of the compressed portions 30, 35 and 36, on the embossed portions 20. The embossed portion 20 is a region extending in a longitudinal direction of the sanitary napkin, and the compressed portions and the non-compressed portions are alternately formed thereon. The embossed portion 20 is formed to be generally linear; however, the width of the first embossed portion is greater than the width of the second embossed portion, and, in the present embodiment, the width of the anterior embossed portion 22 and the posterior embossed portion 23 are substantially equal. The side sheets 6a and 6b are compressed and joined to the surface sheet 2 at the embossed portion 20. In cases where a surface sheet 2 with openings is used, the side sheets 6a and 6b may be joined to the intermediate sheet 5 at the embossed portion.

A length W1 in the width direction (WD) of each of the plurality of compressed portions 35 and 36 constituting the anterior embossed portion 22 and the posterior embossed portion 23 (the first embossed portion 21) is greater than a length W2 in the width direction of each of the plurality of compressed portions 30 constituting the second embossed portion 24. In other words, the embossed portion 20 is formed to have W1 greater than W2 in FIG. 4. Specifically, the size in the width direction of the first embossed portion 21 is preferably in the range of 3.0 to 15 mm, more preferably in the range of 5.0 to 10 mm. The size in the width direction of the second embossed portion 24 is preferably in the range of 1.0 to 10 mm, more preferably in the range of 2.0 to 7.0 mm. It should be noted that, the width of the embossed portion is considered to be the greatest width among a plurality of compressed portions constituting the embossed portion (see the second embodiment described later).

FIG. 4 is an enlarged view of a vicinity of the compressed portion 36 shown in the lower right portion of FIG. 1. Hereinafter, "inner side" designates a central portion in the width direction of the sanitary napkin 1, in other words, the side IS shown in FIG. 4. Similarly, "outer side" designates a side edge side of the sanitary napkin 1, in other words, a side OS shown in FIG. 4.

As shown in FIG. 4, the inner side edge 27 in the width direction of the second embossed portion 24 is formed more outwardly than the inner side edge 26 in the width direction of the posterior embossed portion 23 as the first embossed portion 21. The same applies to the inner side edge in the width direction of the anterior embossed portion 22. This makes the absorbable area of the absorbent body in the central portion B, in which the second embossed portion 24 is formed, greater than the absorbable area in the anterior portion A and the posterior portion C, in which the first embossed portion 21 is formed.

The embossed portion 20 joins the side sheets 6a and 6b with the surface sheet 2, and acts as a folding line of the side sheets 6a and 6b. As described later, the side sheets 6a and 6b are folded in a more outward position in the width direction in the second embossed portion 24, than in the anterior embossed portion 22 and the posterior embossed portion 23 (the first embossed portion 21). The inner ends 6c and 6d of the side sheets 6a and 6b are separated from the surface sheet, and stand up in the thickness direction when a deforming force due to a motion of the wearer's leg is applied thereto, thus inhibiting side leakage. The central portion B in which the second embossed portion 24 is provided is a portion brought into contact with the excretion portion of the wearer and can absorb a greater amount of body fluid such as menstrual blood than the anterior portion A and the posterior portion C in which the first embossed portion 21 is provided.

It should be noted that, the size in the longitudinal direction of the anterior embossed portion 22 and the posterior embossed portion 23 may be altered accordingly in accordance with the intended usage of the sanitary napkin. For example, in a product for days of a regular amount of menstrual blood, the anterior embossed portion 22 and the posterior embossed portion 23 may not have a large difference in size. Contrarily, in a product for use overnight by a wearer lying and a product for days of an increased amount of menstrual blood, the size of the posterio embossed portion 23, which is brought into contact with the buttocks, may be larger than the size of the anterior embossed portion 22.

### 1.5.2 Compressed portion

The area per unit of the compressed portions 35 and 36 in the anterior embossed portion 22 and the posterior embossed portion 23 (the first embossed portion 21) is larger than the area per unit of the compressed portion 30 in the second embossed portion 24.

The ratio between the compressed portion constituting the first embossed portion 21 and the compressed portion constituting the second embossed portion 24 (for example, the ratio between the compressed portions 36 and 30) is in the range of 1.5 : 1 to 5 : 1 (the first embossed portion : the second embossed portion). If the ratio is greater than 5 : 1, the increased area of the compressed portion of the first embossed portion may result in discomfort during wearing. Particularly, the ratio is preferably in the range of 2 : 1 to 4 : 1.

The size in the longitudinal direction of each of the compressed portion constituting the first embossed portion 21 and the compressed portion constituting the second embossed portion 24 (for example, the size in a longitudinal direction of the compressed portions 36 and 30) is preferably in the range of 1.0 to 15 mm, more preferably in the range of 1.5 to 7.0 mm. If the size exceeds 15 mm, stiffness of the compressed portion may inhibit bending and deformation along the wearer's body, thus being undesirable. Additionally, if the size exceeds 15 mm, in cases in which a breakage occurs due to a force applied in a width direction to the first embossed portion, a continuous breakage may occur.

The compressed portions 30, 35 and 36 formed along the longitudinal direction are portions in which each of the side sheets 6a and 6b is joined to the surface sheet 2 by heat embossing. The compressed portions 30, 35 and 36 are formed being spaced apart from each other.

The stiffness of each of the compressed portions 35 and 36 constituting the anterior embossed portion 22 and the posterior embossed portion 23 (the first embossed portion) is lower than a stiffness of each of the compressed portions 30 constituting the second embossed portion 23. This is because, in a manufacturing method embossing the entire surface of FIG. 1 with an equal pressure, the pressure applied to each of the compressed portions depends on the surface area thereof. In other words, a higher embossing pressure is applied to the central portion B of the sanitary napkin 1 having a smaller surface area of compressed portion, compared to the anterior portion A and the posterior portion C having a greater surface area of compressed portion. As a result, the compressed portion 30 of the central portion B has a compression depth greater than the compressed portions 35 and 36.

As each of the compressed portions 30, 35 and 36 is formed along a longitudinal direction of the side sheets 6a and 6b being spaced apart from each other, non-compressed portions 33, 37 and 38 are formed between each of the compressed portions 30, 35 and 36 and the adjacent compressed portions 30, 35 and 36. In other words, compressed portions 30, 35 and 36 and the non-compressed portions 33, 37 and 38 are formed alternately. The stiffness of the compressed portion 30 is higher than the stiffness of the non-compressed portion 33. The sanitary napkin 1 thus can bend more flexibly with the non-compressed portion 33 as a buffer region.

The size in a longitudinal direction of the non-compressed portions constituting the first embossed portion 21 and the non-compressed portions constituting the second embossed portion 24 (for example, the compressed portions 37 and 38) is preferably in the range of 0.5 to 5.0 mm, more preferably in the range of 1.0 to 3.0 mm. If the size is less than 0.5 mm, the ratio of compressed portion is too high and the stiffness of the compressed portion may inhibit bending and deformation along the wearer's body, thus being undesirable. To the contrary, if the size exceeds 5.0 mm, the abovementioned side leakage can easily occur and the joining strength between the side sheets 6a and 6b and the surface sheet 2 is reduced, thus being undesirable.

### 1.5.3 Folding line

As shown in FIG. 2, the inner ends 6c and 6d in a width direction of the side sheets 6a and 6b in the central portion B are disposed so they can be separated from the surface sheet 2. The side sheets 6a and 6b are disposed so that at least a part thereof covers the surface sheet 2 and are joined to the surface sheet 2. The side sheets 6a and 6b are joined to the surface sheet 2 by compressing by a compression method such as heat embossing.

The inner ends 6c and 6d in a width direction of the side sheets 6a and 6b are disposed so as to be spaced apart (separated) from the surface sheet 2. The inner ends 6c and 6d in a width direction of the side sheets 6a and 6b are folded at the compressed portion 30 when the sanitary napkin 1 is worn and the central portion B thereof is deformed by a motion of the wearer's leg. This makes the side sheets 6a and 6b in the central portion B stand up toward a skin contacting side and act as leakage proof walls.

Additionally, even if a large amount of menstrual blood spreads out fully to the width of the surface sheet in the central portion of the sanitary napkin 1, the menstrual blood is prevented from bleeding on the side sheets 6a and 6b separated from the surface sheet 2.

### 1.6 Flap portion

As shown in FIG. 2, the sanitary napkin 1 includes side flaps F1 and F2. The side flaps F1 and F2 are formed by adhering by a hot melt adhesive, a part of the side sheets 6a and 6b in the central portion B projecting outwards in a width direction with a part of the back sheet 3 projecting outwards in a width direction. As shown in FIG. 1, the sanitary napkin 1 is fixed to the underwear by, for example, fastening the side flaps F1 and F2 being folded back to an external surface of the crotch portion of the underwear (not shown) which is a fastening target. This prevents the menstrual blood from leaking from the central portion B due to a disengagement between the sanitary napkin 1 and the underwear, when a compressing force in the width direction is applied from the wearer's thigh. It should be noted that the present invention is not limited to the abovementioned configuration and the side flaps F1 and F2 may not be provided. Additionally, a plurality of side flaps can be provided in the posterior portion C.

As described above, the sanitary napkin 1 can flexibly bend in the longitudinal direction since the compressed portions 30, 35 and 36 are formed discontinuously in the embossed portion 20. This is because, when the sanitary napkin 1 bends along the curvature of the wearer's body, border regions between the high-stiffness compressed regions 30, 35 and 36 and the low-stiffness non-compressed regions 33, 37 and 38 act as folding lines, and the low-stiffness non-compressed regions 33, 37 and 38 act as buffer regions, allowing the sanitary napkin 1 to bend flexibly along the embossed portion 20. Particularly, when the sanitary napkin 1 is worn, a deforming force due to a motion of wearer's legs is greater in the central portion B than in the abdomen side and in the back side. Therefore, if the second embossed portion 24 can deform more flexibly, discomfort against the wearer's body can be reduced. It should be noted that, in the present invention, the compressed portion 30 in the central portion B may be the only compressed portion formed discontinuously.

Generally, an embossed portion formed continuously on a non-woven fabric and the like makes the fiber density of the embossed portion high. If a body fluid such as menstrual blood adheres to a high-density region, the blood may be easily dispersed by a capillary effect. In this respect, the high density region in the embossed portion is discontinuous in the present embodiment. This prevents the menstrual blood from being dispersed by the capillary effect and the wide dispersion of the menstrual blood. Thus, the appearance does not provide the impression to the wearer that leakage has occurred.

Absorbent articles such as sanitary napkins fixed to an article of underwear are subjected to a deforming force due to a motion such as walking, standing, and sitting. Among others, a portion disposed along the buttocks is repeatedly compressed by a motion of sitting, and a delamination force is applied thereto. To be resistant to this force, the first embossed portion 21 is preferably more strongly joined than the second embossed portion 24. The width of the first embossed portion 21 is greater than that of the second embossed portion 24, and the area per unit of the first embossed portion 21 is greater than that of the second embossed portion 24. Therefore, the side sheets 6a and 6b are more strongly joined to the surface sheet 2 in the first embossed portion. This can prevent breakages during wearing and a leakage of the body fluid from a breakage.

Furthermore, to obtain the area per unit of the first embossed portion 21 greater than that of the second embossed portion 24, the widths W1 and W2 are differentiated and the second embossed portion 24 is formed more outward in the width direction than the first embossed portion 21. Thus, the absorbable area in the central portion B in which the second embossed portion 24 is provided becomes greater than in the anterior portion A and the posterior portion C in which the first embossed portion 21 is provided.

### 2. Second Embodiment

A second embodiment of the present invention will be described below, with reference to FIGS. 5 to 7. In the following description, the same reference numerals have been retained for similar parts that are identical to those described in the first embodiment, with the description thereof omitted.

As shown in FIG. 5, the sanitary napkin 1A according to the second embodiment is different from the first embodiment in respect of the embossing pattern. FIG. 6 is an enlarged view of the first embossed portion according to the second embodiment; FIG. 7 is an enlarged view of the second embossed portion according to the second embodiment.

As shown in FIG. 5, the first embossed portion 21A includes the first compressed portion 31A and 31C and the first auxiliary compressed portion 32A and 32C. Specifically, the first compressed portion 31A and the first auxiliary compressed portion 32A are formed alternately in the anterior embossed portion 22A (the first embossed portion 21A), and the first compressed portion 31C and the first auxiliary compressed portion 32C are formed alternately in the posterior embossed portion 23A (the first embossed portion 21A). The second embossed portion 24A includes the second compressed portions 30A and 30B.

As shown in FIG. 6, the width of the anterior embossed portion 22A and the posterior embossed portion 23A is greater than the width of the second embossed portion 24A. In other words, the embossed portion 20 is formed to have W3 greater than W5 in FIG. 6. In the present invention, size in a width direction of the embossed portions is compared in view of the maximum width thereof. Specifically, in the present embodiment, the maximum width, constituted of the width of the first compressed portion 31A (not shown) and the width of the first auxiliary compressed portion 32A (W4), is the length in the width direction W3 of the first embossed portion 21. Similarly, the maximum width, constituted of the second compressed portions 30A and 30B is the length in the width direction W5 of the second embossed portion 24A.

The first compressed portion 31A and 31C and the first auxiliary compressed portion 32A and 32C may be a collective unit of compressed portions 31a and not necessarily a continuous area.

The second embodiment, in which the first compressed portions 31A and 31C and the first auxiliary compressed portions 32A and 32C are alternately disposed with different widths, can be bent more flexibly than the first embodiment in which the compressed portions 30, 35 and 36 are formed with an equal width. This is because the surface area of the non-compressed portions 33, 37 and 38 between the first compressed portions 31A and 31C and the first auxiliary compressed portions 32A and 32C is greater than in the first embodiment. Additionally, since the first compressed portions 31A and 31C and the first auxiliary compressed portions 32A and 32C are collective units of compressed portions, a discontinuous compressed portion can be obtained and thus the sanitary napkin 1A can bend flexibly in a longitudinal direction.

As shown in FIG. 7, each of the plurality of compressed portions 30A constituting the second embossed portion 24A has a crossover region S1 with neighboring compressed portions 30A and 30B, when viewed in a width direction. The crossover region S1 is preferably greater than 0.5 mm and less than 7.0 mm. The distance between the neighboring compressed portions 30A and 30B is selected accordingly in accordance with the shape and the arrangement thereof; however, the minimum distance is preferably in the range of 0.5 to 5.0 mm, more preferably in the range of 1.0 to 2.5 mm.

The area per unit of the compressed portions 30A and 30B in the second embossed portion 24A is less than the area per unit of the first compressed portions 31A and 31C and the first auxiliary compressed portions 32A and 32C in the first embossed portion 21A, however, with the abovementioned configuration, the side sheets 6a and 6b can be strongly joined to the surface sheet 2. On the other hand, the compressed portions 30A and 30B disposed discontinuously provide flexibility. It should be noted that, the abovementioned crossover region is preferably provided not only in the second embossed portion 24A, but also in the first embossed portion 21A. Specifically, a crossover region S2 is provided also between the the first compressed portion 31A and the first auxiliary compressed portion 32A, which brings the similar effect as in the abovementioned second embossed portion 24A.

In the second embodiment, collective units of compressed portions 31a are formed into the first compressed portion 31A and 31C in a pattern of five petal flowers, and the first auxiliary compressed portions 32A and 32C are formed in a pattern of leaves. Each of the petals is formed in a substantially oval shape or a teardrop shape. The long axis of the oval shape or the teardrop shape is arranged radially to form a substantial pentagon with a space in the center thereof to form a flower shape. The leaf is formed in a substantially oval shape or a teardrop shape, in which the longitudinal direction thereof is substantially parallel to the longitudinal direction of the sanitary napkin. The pattern is not limited thereto as long as the first compressed portions 31A and 31C and the first auxiliary compressed portions 32A and 32C with different widths are alternately formed; the first auxiliary compressed portions 32A and 32C may also be a collective unit of other compressed portions. Since the pattern is a collective unit of fine compressed portions as shown in FIG. 7, the non-compressed portions 37 and 38 between the compressed portions can absorb forces in various directions when the sanitary napkin 1 bends. This can allow the sanitary napkin 1 to bend more flexibly.

Additionally, according to the pattern of the second embodiment, the first auxiliary compressed portions 32A and 32C formed in a leaf-like shape are formed in the inner ends 6c and 6d of the side sheets 6a and 6b. In the anterior portion A and the posterior portion C of the sanitary napkin, the side sheets 6a and 6b are joined to the surface sheet 2 so as not to be separated therefrom. In a case where a deforming force due to the wearer's motion is applied during wearing, the leaf-shaped first auxiliary compressed portions 32A and 32C can prevent the delamination of the inner ends 6a and 6d of the side sheets from the surface sheet 2.

Additionally, the wearer in the menstrual period may experience physical discomfort, or may be disturbed by looking at the menstrual blood while changing the sanitary napkin 1 for a new one. The embossed portion 20A formed in the refreshing flowers and leaves can comfort the wearer while changing the sanitary napkin 1A for a new and clean one.

### 3. Others

It should be noted that, as shown in FIG. 8, the embossed portion 20 is not limited to a particular shape as long as the area per unit of the first embossed portion 210 is greater than that of the second embossed portion 240. For example, the compressed portions 360, constituting the anterior embossed portion (not shown) being the first embossed portion 210 and the posterior embossed portion 230, may be spread in a width direction as shown in FIG. 8. As the surface area of the embossed portion increases, the stiffness also increases proportionately and may result in a discomfort during wearing. The compressed portions 360 spread in a width direction while having the same area per unit as in the first embossed portion 21 shown in FIG. 1, constituting the first embossed portion 210, can maintain flexibility without increasing the stiffness.

## Claims

1. An elongated shaped absorbent article, comprising:
a surface sheet which is at least partially liquid permeable;
a back sheet which is liquid impermeable;
a liquid retentive absorbent body which is disposed between the surface sheet and the back sheet;
a pair of side sheets which is disposed on both sides of the absorbent body so that at least a part thereof covers the surface sheet; and
an embossed portion having a plurality of compressed portions formed along the pair of side sheets,
wherein the embossed portion has a first embossing portion which is formed in both edge portions in a longitudinal direction of the absorbent article, on a front edge side and a rear edge side, and a second embossing portion which is formed in a central portion in the longitudinal direction,
the first embossed portion has a width in a width direction orthogonal to the longitudinal direction greater than a width of the second embossed portion, and
compressed portions constituting the second embossed portion are formed so as to be spaced apart from each other.

2. The absorbent article according to claim 1,
wherein the second embossed portion has the plurality of compressed portions and a plurality of non-compressed portions formed between each of the compressed portions formed so as to be spaced apart from each other, and
a stiffness of each of the plurality of compressed portions is greater than a stiffness of the plurality of non-compressed portions.

3. The absorbent article according to claims 1 or 2,
wherein each of the plurality of compressed portions constituting the second embossed portion has a crossover region in the width direction with a neighboring compressed portion.

4. The absorbent article according to any one of claims 1 to 3,
wherein an inner edge in the width direction of the second embossed portion is formed outside of an inner edge in the width direction of the first embossed portion.

5. The absorbent article according to any one of claims 1 to 4,
wherein a stiffness of each of compressed portions constituting the first embossed portion is lower than a stiffness of each of compressed portions constituting the second embossed portion.

6. The absorbent article according to any one of claims 1 to 5,
wherein a pressure for forming each of a plurality of compressed portions constituting the first embossed portion is lower than a pressure for forming each of a plurality of compressed portions constituting the second embossed portion.

7. The absorbent article according to any one of claims 1 to 6,
wherein the first embossed portion has:
a plurality of first compressed portions of a length in the width direction substantially same as a length in the width direction of the first embossed portion; and
a first auxiliary compressed portion of a length in the width direction shorter than a length in the width direction of the first compressed portion, and
the first compressed portion and the first auxiliary compressed portion are formed alternately in the longitudinal direction.

8. The absorbent article according to any one of claims 1 to 7,
wherein the embossed portion joins the side sheet to the surface sheet.

9. The absorbent article according to any one of claims 1 to 8,
wherein, in a region formed in the second embossed portion in the longitudinal direction, an inner edge in the width direction of the side sheet is configured to be separable from the surface sheet, and
the second embossed portion acts as a folding line for separating the inner edge of the side sheet from the surface sheet.
